# EUROPEAN PATENT SPECIFICATION

(11) **EP 3 860 392 B1**
(45) Date of publication and mention of the grant of the patent: **07.06.2023**
(21) Application number: 19774125.9
(22) Date of filing: 01.10.2019
(51) Int. Cl.: A45D 19/00, A61K 8/26, A61Q 5/00, A61Q 19/00, A61K 8/96, A61K 8/20

(54) **COSMETIC TREATMENT PROCESS**
VERFAHREN ZUR KOSMETISCHEN BEHANDLUNG
PROCÉDÉ DE TRAITEMENT COSMÉTIQUE

(30) Priority: 02.10.2018 FR 1859106
(43) Date of publication of application: 11.08.2021
(73) Proprietor: L'OREAL, 75008 Paris (FR)
(72) Inventor: SAMAIN, Henri, 94152 Chevilly Larue (FR); GIRON, Franck, 94152 Chevilly Larue (FR); ROBINAULT, Jean-Luc, 93400 Saint-Ouen (FR)
(74) Representative: Cabinet Nony
(86) International application number: PCT/EP2019/076634
(87) International publication number: WO 2020/070153

(56) References cited:
- FR-A1- 2 931 644

## Description

The present invention relates to skin or hair treatment processes and devices.

### Prior art

In several situations, it is desired to carry out an abrasive action on the skin or the hair.

With regard to the scalp, it is desired for example:
- to remove skin fragments that are dead or not attached to the scalp, for the purposes of softening or so as to avoid them falling onto clothing in the form of dandruff,
- to bring about a reaction of the scalp, a phenomenon that is liked by many individuals since it gives a sensation of vitality.

With regard to the hair, it is desired for example:
- to abrade the surface thereof in order to bring a softness back to the lengths of the hair, in particular damaged hair;
- to sensitize the surface of natural hair (and not chemically treated) by abrasion in order to improve treatment performance levels.

The methods used at the current time come under four approaches:
a) the chemical route, by means of acids such as hydroxy acids, which can soften the material of the skin or of the hair;
b) the mechanical route, termed "scrub" way, by means of particles which are made to roll and which bring an abrasive mechanical effect;
c) the biological route, by means of UV radiation for example, which disrupts the cells, and can bring about accelerated exfoliation of the skin;
d) the microbiological route, by means of the regulation of the microbiome, which can bring about a reaction of the skin.

The chemical way is limited because it can, in certain individuals, lead to adverse reactions, and the result is not equal from one individual to the other. Thus, it is difficult to control.

The mechanical route is also limited:
1) while the step of applying an abrasive powder to the hair is already not something that is easy, the mechanical action required to exploit the abrasive properties of the powder is even more difficult to perform. It is necessary to rub the hairs against one another, which makes the operation firstly lengthy, and especially random. Logically, some parts of the head of hair receive too much rubbing and others not enough. In several cases, it is observed that hairs are damaged with, according to examination under an electron microscope, scratches along the fibres, which is a sign that the abrasion has not produced the desired effect.
   This lack of control of the effect can admittedly be improved if the process is performed little by little. However, to control the effect, the user must rinse the hair before evaluating the result, since, before rinsing, impaired by the presence of the abrasive, said user cannot discern the quality of the hair. This procedure is thus very lengthy and poorly compatible with commercial use.
2) In the case of the skin, the application of an abrasive powder is rather easy, in particular by means of thickened compositions. The process requires rubbing the composition against the skin using the hand or by means of an object for example. Several defects are observed: once the abrasive powder composition has been applied, there is a tendency to move the powder as the rubbing proceeds. The feeling of abrasion informs the user whether or not powder remains in the area of interest, but the feeling is quickly lost, which makes it difficult to properly control the treatment. Thus, the user no longer clearly knows which areas are treated and which areas are not treated. The problem is exacerbated when the individual carrying out the treatment (for example a hairstylist) is not the individual undergoing the treatment. It is then necessary for said hairstylist to ask the individual how it feels. Another defect is that it is painstaking to treat large surface areas since the application of the thickened composition takes time, as does the rubbing. Another problem is linked to the difficulty in treating certain areas, such as the back for example, since it requires even more time and attention. Thus, the scrub way is instead limited to small areas, such as for example the face for the treatment or prevention of acne in particular. Thickening of the abrasive composition is required in order to enable it to be applied, otherwise the composition would run and would not remain on the area of skin to be treated. The compositions used are often referred to as "shower scrubs", in the sense that it is recommended to use them while showering, but also because the compositions contain surfactants capable of improving the exfoliation treatment effect and the removal of dead skin.

Application FR 2 931 665 describes a hair abrasion process.

Application FR 2 931 644 discloses a process for treating the hair, in which abrasive particles are projected onto the head of hair by a vector fluid. In the examples given in this application, this vector fluid is a gas. This can pose a problem of generation of noise and dust, which is detrimental to the comfort of the individual and their environment and can require the setting up of a confinement chamber and suction systems for preventing the particles from coming into contact with the respiratory tracts.

3) The abrasive powders route also poses the problem of the particle size, both in the case of hair treatment and in the case of skin treatment. If the powders have large particle sizes (100 µm or more), the rubbing is quite easy but the particles roll rather than abrading. If much finer powders (1 µm-10 µm) are chosen, the rubbing is made difficult, producing a placing effect. The thickening of the compositions plays a positive role for helping to produce the movement, but also a negative role since it brings a lubricating effect which does not help the attachment of the particles and thus limits the abrasion performance level.

The biological and microbiological route pose, like the chemical route, problems of potential skin reaction.

### Summary

There thus remains at the current time a problem that has not been entirely satisfactorily solved for in particular:
a) carrying out an efficient abrasive treatment on the skin and the hair,
b) without unduly damaging the condition of the hair, in particular not producing scratches along the hairs,
c) in a practical manner and in particular:
   a. rapidly,
   b. without requiring the use of the hands or rubbing,
   c. and applicable both to small surface areas and to large surface areas; and
d) without requiring the use of potentially irritant chemical substances such as acids, or surfactants.

The invention aims to satisfy all or some of the problems mentioned above and it achieves this by virtue of a process according to claim 1 and a device according to claim 12.

The use of a flow of composition under pressure and at low flow rate and containing a pulverulent compound makes it possible to obtain an abrasion effect on the hair and/or on the skin without the drawbacks described above.

In particular:
1) the application is rapid and practical,
2) it does not require rubbing the hair or the skin,
3) it is applicable to large surface areas; thus, it is possible to treat the entire body in less than one minute, or the entire head of hair in less than 30 seconds.

Furthermore, the invention makes it possible to limit the generation of dust and noise. In so doing, the invention enables a use in the form of a treatment course or a milder and repeated application.

The invention makes it possible to use abrasive powders having particles of very large sizes (300 µm or more), of large sizes (50 µm -300 µm), but also of small sizes (10 µm or less) depending on whether it is desired to create a stimulation effect, to rid the surface of pieces (such as dandruff or edges of flaking) or to create a surface sensitization.

The application possibilities are in particular:
- the treatment of damaged hair, to remove the edges of flaking and to improve the sheen;
- the treatment of natural hair in order to sensitize the surface thereof;
- the treatment of greasy hair and scalps in order to limit regreasing, and dandruff;
- the stimulation of the skin and/or of the scalp for the purposes of massaging and relaxing;
- the treatment of the skin in order to remove dead skin in general (over the entire body) or to treat specific areas, for example areas where the skin is thick;
- the cosmetic treatment of healthy areas of acne-prone skin.

Furthermore, it is observed that the invention can be used for the body. The invention is therefore especially advantageous for treating areas that are difficult to access, such as the back, the back of the neck, the back of the legs and, for individuals who are not flexible, the feet or the armpits.

The use of rubbing is not required. However, in order to reinforce the effect and the impression of action, it is possible to perform massaging before, during or after the treatment.

Preferably, the invention uses water or an aqueous composition as vector liquid. Thus, the composition preferably comprises water, in particular is predominantly constituted of water (by weight). The composition may comprise only water and the solid particles, or, as a variant, water supplemented with certain additives, in particular at least one care active agent or a washing agent, and the solid particles.

The flow rate is preferably between 0.4 L/min and 4 L/min, better still between 1 and 4 L/min. A flow of composition delivered by the device of greater than or equal to 0.4 L/min allows good penetration of the composition into the hair. If the flow rate is less than 0.4 L/min, the penetration of the composition may be insufficient, and an accumulation of particles is obtained at the surface while the vector fluid, preferably water, flows on the head of hair. This accumulation is then difficult to remove. Above a flow rate of 10 L/min, defects re-emerge: the vector fluid causes the head of hair to swell and creates an accumulation which causes an obstacle to the impact of the particles, reducing the speed of the droplets and the effect. Furthermore, large amounts of liquid flow and are wasted.

The composition is preferably delivered under a pressure of 6 bar or more, preferentially greater than 8 bar. The pressure is measured upstream of the nozzle(s).

The pressure is preferably between 6 and 20 bar, even better still between 8 and 20 bar. The orifices have for example a circular cross-section and/or a diameter between 0.1 and 2 mm, a cross-section sufficient to allow the abrasive particles to pass through.

The droplet speed contributes to the abrasion or stimulation effect. The best abrasion results are obtained for a pressure of more than 4 bar and a droplet speed of at least 8 m/s and preferably greater than 15 m/s. Such a droplet speed makes it possible to rapidly produce the desired abrasion effect. The droplet speed preferentially remains less than 50 m/s. Particularly good results are obtained around a pressure of 6 to 20 bar, a flow rate of 1 to 4 L/min, and a weight concentration of solid particles of 0.5% to 5% for a repeated treatment and of 5% to 25% for a single treatment. The term "repeated" treatment should be understood to mean that the frequency of the treatment is greater than or equal to once a month. The term "single" treatment itself is less than or equal to once a month.

The flow is generated from the collision of at least two jets generated by nozzles oriented with an angle of between 30 and 120° with respect to one another, preferably of about 90°. The collision of the jets makes it possible to transfer this kinetics energy to reduction of the droplet size, while at the same time ensuring a high jet speed. This produces a resulting flow that is wetting while at the same time having a low water consumption; it is advantageous to have a low water consumption because this makes it possible to have a notable concentration of particles in the flow and a possibility of controlling this concentration. In this way, the product losses are also reduced.

The solid particles are preferably present in the composition upstream of the nozzle(s) in a concentration by weight of greater than or equal to 0.5% relative to the total weight of the composition, better still between 0.5% and 25%.

Preferably, the particles have a hardness on the Mohs scale of greater than or equal to 3. The particles can be chosen in particular from powders of alumina (Mohs=8), of silica (Mohs=7), of aluminosilicates (Mohs=7), of carbonates (Mohs=3), or of a material coated with a silica, an alumina or an aluminosilicate, and mixtures thereof.

The particle speed at the moment they impact the surface to be treated can be greater than or equal to 8 m/s and preferably greater than 15 m/s. This surface can be a surface of the hair, the treatment then aiming for example to remove the edges of flaking and to improve the sheen of the hair, to sensitize the surface of the hair, and/or to degrease the hair.

This surface may also be a skin surface, in particular a scalp surface, the treatment aiming in particular to exfoliate or remove the dandruff, to stimulate the skin, in particular of the scalp, for the purposes of massaging or relaxing, and/or to treat acne-prone healthy skin.

The particle size may be between 0.1 and 500 microns, in particular between 0.1 and 50 microns for treatment of the hair, in particular for softening damaged hair, and between 10 microns and 300 microns, for treatment of the scalp, in particular for reducing the problems of dandruff or for activating the scalp. In the case where it is desired to treat the hair and the scalp, the particle sizes can be mixed or else particles of 10 to 100 microns can be chosen.

The process can comprise the step consisting in rinsing said surface with the same device as that used for projecting the composition, by projecting water without said solid particles and/or water with a surfactant or a compound capable of dissolving the particles.

In one exemplary embodiment, the solid particles are suspended by mechanical stirring in a reservoir and they are left to settle, and, during the settling, the composition is suctioned from this reservoir so as to allow the nature and/or the concentration of the particles present in the flow distributed to vary over time during the dispensing, due to the settling out taking place in the reservoir. For example, it is possible to obtain in this way a dispensing of composition with a concentration of large particles which decreases over time. The taking of the composition from the reservoir can be carried out at a certain distance from the bottom so as not to take the particles which accumulate in the bottom. The volume of the reservoir can be sufficiently large to make it possible to deliver the volume of composition required for the treatment while at the same time allowing this accumulation of particles in a "dead" volume, not taken, in the bottom of the reservoir.

It can also be envisaged to produce, by mixing and mechanical stirring, a suspension of particles having different densities, which settle at different speeds, thereby making it possible to have a change, during the treatment, of the formulation of the composition dispensed, and in particular a change in the weight fraction of a certain category of particles relative to another, of different density.

The surface to be treated may be dry before the beginning of the treatment. The process is preferentially carried out on dry hair, but can also be done on wet hair. The process is preferentially carried out on dry skin, but can also be done on wet skin.

A subject of the invention is also a device according to claim 12.

The device advantageously comprises a pump connected to said at least one nozzle. This pump can be supplied by running water for example or by a reservoir containing the composition. It may be a centrifugal pump.

Since the dispensing device comprises two nozzles oriented such that their jets encounter one another, the axes of the nozzles make an angle between them of between 60° and 120°, in particular of about 90°.

The device may comprise a stirrer for suspending said particles in the reservoir. In particular, the device may be arranged to remove the composition to be dispensed by suctioning from this reservoir, the suctioning being carried out at a distance from the bottom of the reservoir.

The device can comprise two reservoirs containing compounds which react together, and a system for injecting these compounds so as to allow them to mix with the vector liquid and to react, the reaction of the compounds producing solid particles.

In one particular embodiment of the invention, the device is arranged to send water under pressure with the solid particles, then automatically or by triggering by the user:
- water without powder
   or
- water with an ingredient capable of removing the material of the powder, such as a surfactant.

The device can thus comprise several reservoirs containing different compounds intended to be delivered at various stages of the treatment, for example a reservoir of an abrasive powder and at least one reservoir of a washing agent.

It is also possible to apply a composition containing an active agent capable of dissolving the powder. Thus, in the case of a powder composed of a carbonate, a composition containing an acid, which will allow dissolution of the powder, can be applied. Rinsing can end the treatment. In this case, the device can comprise a reservoir of an abrasive powder and at least one reservoir of an agent for dissolving this powder, in particular an acid solution.

### Particles

The solid particles according to the invention can be composed of a pulverulent material that is water-insoluble (silica, alumina, carbides, etc.) or has very low water solubility (calcium carbonate, calcium phosphate, etc.) or is water-soluble (NaCl, KCl, etc.). The latter possibility is especially advantageous for aiding the final removal.

For the purposes of the present invention, the term "water-insoluble" is intended to mean a compound of which the solubility at spontaneous pH in water at 25°C and at atmospheric pressure is less than 0.1%.

For the purposes of the present invention, the term "water-soluble" is intended to mean a compound of which the solubility at spontaneous pH in water at 25°C and at atmospheric pressure is greater than 1%, that is to say forming at this concentration a macroscopically homogeneous, transparent and isotropic medium.

The solid particles may be grains of NaCl or of another mineral salt, that will partially dissolve during their injection in the flow of water and will reach the hair in the form of particles; it will be possible for these particles to then be removed with water.

The particles are preferably abrasive solid particles. The term "abrasive solid particles" should be understood to mean particles having a hardness greater than or equal to that of the hair. For example, the abrasive solid particles can have a hardness greater than or equal to 3 on the Mohs scale, or even greater than or equal to 4, for example greater than or equal to 5 on the Mohs scale.

The solid particles can be chosen from natural materials, in particular of mineral or plant origin, or synthetic materials. The abrasive solid particles can for example be chosen from the following materials, this list not being limiting:
- inorganic and/or metal particles such as boron nitride, in cubic form (Borazon^{®}), aluminosilicate, zircon, mixed aluminium oxides such as emery, zinc oxide, aluminium oxides such as aluminas or corundum, titanium oxide, titanium oxide-coated mica, carbides, in particular silicon carbide (carborundum), or the other metal oxides, metals and metal alloys such as iron shot, steel shot, in particular perlite; silicates such as glass, quartz or sand, calcium carbonate (for example Bora Bora sand or pink marble) or magnesium carbonate, pumice stone, amorphous silica, diamond, ceramics,
- organic particles such as the stone of fruit, in particular the stone of apricots, for example the Scrubami^{®} apricot, wood cellulose, for example ground bamboo cane, coconut shell, for example Coconut exfoliator; polyamide, in particular Nylon-6, and
- mixed particles combining organic and inorganic compounds, and particles coated with the compounds described above.

The solid particles can have a flattened, spherical, elongated, polyhedral or irregular shape. The solid particles can originate for example from grains of pumice stone powder, of diamond powder, of fruit stone powder, of coconut shell powder, from microbeads, for example microbeads of alumina, of glass, of polyamide, in particular of Nylon-6, or from fibres, in particular polyamide fibres, hardwood cellulose fibres.

An especially advantageous variant consists in using particles having a density of less than 1 g/cm³, preferably being chosen from cellulose aerogels and powders. These powders of density less than that of water may be easily harvested at the end of treatment by bringing the hair into contact with water. It will then be sufficient to bathe the hair in a volume of water, then to move the hair and to harvest the powder which begins to float because of its low density.

The following implementations are recommended:
a) the powder is introduced at the time of use into a reservoir which either serves to supply the pump, or serves as an injection reservoir,
b) or else the powder is placed in a solvent in which it is not soluble (for example ethanol for a powder composed of NaCl salt). The device is then used to inject this fluid into the flow of water. The particles will mix with the water while at the same time keeping their particulate form due to the fact that they will not have time to dissolve.

The powder can also be formed in situ by reaction or interaction of two compounds, for example an ammonium carbonate and a calcium chloride. These two compounds will interact together in the flow of water, rapidly forming a calcium carbonate powder.

The powders used can have various sizes. For example, a powder having two size populations such as 1 µm and 50 µm is used to obtain an effect of fine abrasion of the surface and to rid said surface of the pieces in the process of detaching. The abovementioned sedimentation process can be carried out in order to first send the largest particles, then the finest particles, before sending water. It is also possible, by sedimentation, to first send the high-density powders (therefore the most abrasive), then the low-density powders (softer), then water. The low-density powder is for example calcium carbonate, and the higher density powder is a clay powder.

It is possible to mix particles of different densities, having a density difference ΔD of at least one factor such that ΔD = ABSOLUTE VALUE [(DENSITY 1 - DENSITY 2)/( ½ x (DENSITY 1+DENSITY 2))] that is to say greater than 15%, preferably greater than 25%, in one and the same reservoir and to deliver the mixture in the process of settling out, so as to cause the characteristics of the particles present in the flow to vary. The particles of different densities have for example sizes which differ by a factor of at least 2, better still at least 5, even better still at least 10. The particles of different densities can have hardnesses which differ by at least one unit on the Mohs scale. The concentration of particles of a given density is for example equal to from 1 to 10 that of the particles of another density.

### Application of a treatment product

The process according to the invention may comprise the step consisting in applying, for example before or after the abrasion treatment, another treatment product to the hair. The treatment product may be, for example, a cosmetic product, in particular a conditioner, a permanent-waving product, a relaxing product or a product for dyeing or bleaching the hair.

The treatment product may be chosen, for example, from the following products, this list not being limiting:
- products for modifying the mechanical properties of the hair, especially comprising a reducing agent, such as thioglycolic acid and derivatives thereof, cysteine, sulfite, sodium hydroxide, guanidine carbonate, trihydroxymethylphosphine, or an oxidizing agent, such as H₂O₂ or persulfate;
- emollient or penetrant products, comprising, for example, a solvent, a glycol, a plasticizer or a cationic, anionic or amphoteric surfactant;
- products that modify the surface properties of the hair, especially comprising a silicone, a reactive aminosilicone, an adhesive polymer, a non-silicone lubricant comprising fatty substances chosen from plant oils, mineral oils, synthetic oils, and waxes, especially fatty alcohols or fatty esters; and
- products for restructuring the interior of the hair, comprising, for example, an ionene, a protein, a hydroxy acid or a reactive compound, especially a formaldehyde generator, a silane;
- direct dyes or oxidation dyes.

The treatment product may be applied before the abrasion and may contribute towards protecting the hair during the abrasion, in order to avoid excessive abrasion.

The treatment product can form a sheath on the hair. The abrasion can remove all or part of this sheath, which is smoothed on the hair. The material forming the sheath can remain on the recessed reliefs of the hair, for example after the abrasion.

The treatment product is preferably applied after abrasion.

It is possible to apply a first treatment product before abrasion, in particular a reducing product, and a second treatment product after abrasion, in particular an oxidizing product, with the aim for example of modifying in a long-lasting manner the texture or the feel of the hair.

The invention may be understood more clearly from reading the following detailed description of non-limiting exemplary embodiments thereof and from studying the appended drawing, in which:
- Figure 1 is a partial schematic representation of an example of a device according to the invention,
- Figures 2 and 3 are views similar to Figure 1 of implementation variants, and
- Figure 4 shows an example of arrangement of the nozzles within the hand piece.

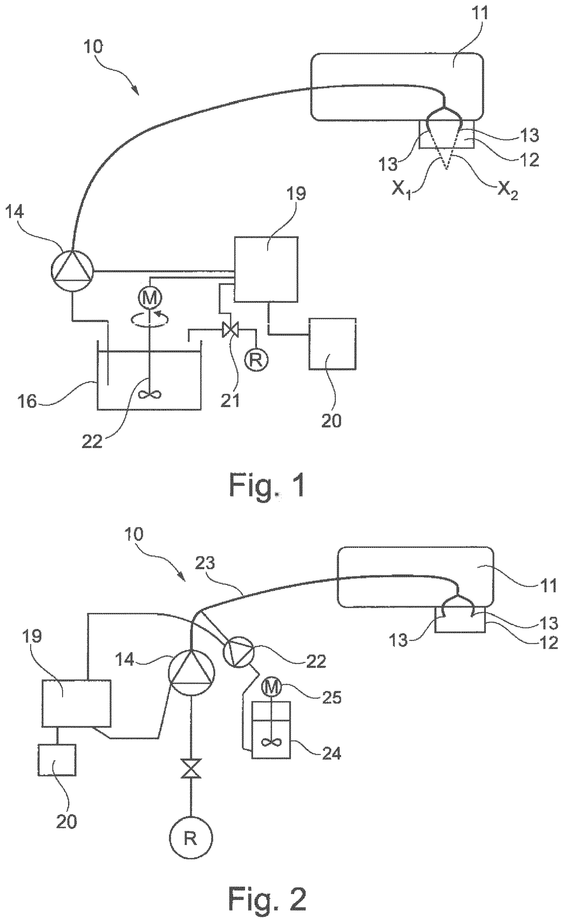

A treatment device 10 according to the invention, comprising a hand piece 11, also referred to as low-flow showerhead, for delivering, onto the surface to be treated, a composition comprising solid particles, in accordance with the invention, has been represented in Figure 1. The solid particles are for example particles having a hardness greater than or equal to 3 on the Mohs scale, for example an alumina powder, a silica powder, an aluminosilicate powder or a carbonate powder or any other matter covered with a hard material such as silica, alumina or aluminosilicate.

The hand piece 11 preferably comprises, as illustrated, a dispensing head 12 having at least two nozzles 13 each delivering a respective jet under pressure.

The X₁ and X₂ axes of the nozzles 13 are oriented such that their jets collide, thereby making it possible to reduce the size of the droplets. The nozzles 13 allow the drops to reach a small size and a speed preferably greater than 8 m/s and preferably greater than 15 m/s. The surface treated by the flow emitted while keeping the hand piece immobile ranges for example from 10 to 100 cm².

Such an arrangement of nozzles is described in patent EP 1 954 893 B1 from the company Creaholic S.A. and can be reproduced. The angle between the X₁ and X₂ axes is for example about 90°.

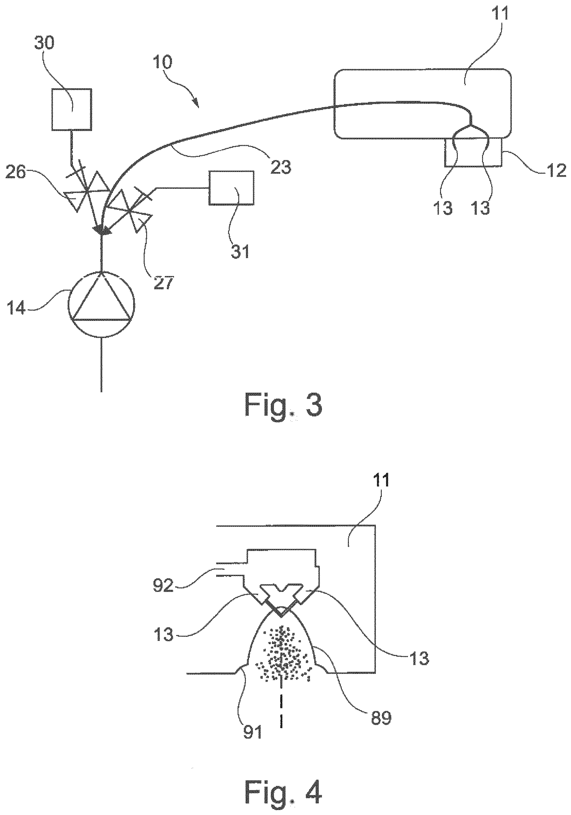

The hand piece 11 has a cavity 89 opened to the outside, within which the jets emitted by the nozzles 13 encounter one another, as illustrated in Figure 4. This cavity 89 can diverge towards the outside, with a recess 91 further widening it in proximity to its opening. The cavity 89 can participate in guiding the flow generated after the encounter of the jets in the axis of said cavity, towards the outside.

The speed of the drops, measured at the opening of the cavity 89, in the axis thereof, can be greater than or equal to 8 m/s.

The device 10 operates in this example with a supply of composition from a reservoir 16.

The device 10 can comprise a pump 14 for raising the pressure upstream of the nozzles 13 to a value greater than or equal to 4 bar. The pump 14 is for example a centrifugal pump. As a variant, the composition is pressurized by other means, such as for example the use of a sufficient level change between the reservoir 16 and the hand piece 11.

An electronic control system 19, for example comprising a microcontroller, is advantageously provided for controlling the operation of the various constituent elements of the device 10, and in particular obtaining, at the outlet, the desired concentration of solid particles and the desired flow characteristics.

The control system 19 can comprise, where appropriate, a man-machine interface 20 making it possible to regulate various operating parameters.

The device 10 can comprise, as illustrated, at least one solenoid valve 21 which makes it possible to open or close the water supply from a network of running water R for example.

The flow of water dispensed has, in the example under consideration, a flow rate of less than 10 L/min, and preferentially of between 0.4 L/min and 4 L/min. The solid particles are preferably present in the water at a concentration by weight of 1% or more, and preferentially between 4% and 30%.

The reservoir may be equipped with a stirrer 22, which can optionally be activated throughout the treatment, depending for example on whether it is desired to vary by settling out the concentration of solid particles during the treatment.

One or more filters 92 can be placed upstream of the nozzles 13 in order to prevent particle agglomerates passing through and blocking the nozzles, as illustrated in Figure 4.

In the example of Figure 1, the solid particles are introduced into the supply of the pump 14 upstream thereof, namely into the reservoir 16.

It is also possible to supply solid particles by adding to the water supply of the pump, as illustrated in Figure 2.

In this case, it is possible to use, as illustrated, an additional pump 22 for injecting, into the water circuit 23 downstream of the pump 14, a powder or a liquid composition containing the solid particles, contained in a reservoir 24. The injection into the water circuit 23 leads to a dilution, which is preferentially (by weight) by a factor of 100 to 1.5, and more preferentially from 10 to 2.

In the example illustrated in Figure 2, the injection of the solid particles takes place downstream of the pump 14 in order to reduce the wear thereof. This injection can also be carried out upstream of the pump 14, which makes it possible to reduce the pressure required to carry out the injection.

In Figure 2, the reservoir 24 equipped with a stirrer 25 for stirring its content has been represented.

In one exemplary embodiment applicable both to the example of Figure 1 and to that of Figure 2, the reservoir 16 or 24 containing a liquid, for example water, is filled with a powder such as a fine sand for example. Stirring is firstly carried out, and the stirring is interrupted. The liquid which is suctioned and sent in the flow and thus onto the hair then contains powder. Then, naturally, the powder sediments in the reservoir 16 or 24 and the liquid which is suctioned becomes clear, then making it possible to automatically carry out a rinsing operation.

In the case where the particles are water-soluble, they can be taken from a reservoir where they are in suspension in a liquid in which they are not soluble, this liquid being for example ethanol in the case of a powder of a mineral salt, for example NaCl. In this case, the particles do not have time to entirely dissolve before reaching the surface onto which they are projected.

In one implementation variant, illustrated in Figure 3, the particles are generated at the time of use by bringing together two compounds which react together to form solid particles, these compounds being in particular injected into the water circuit 23 supplying the hand piece 11, upstream of the pump 14.

In Figure 3, two solenoid valves 26 and 27 which make it possible to control the injection of these two compounds, contained in respective reservoirs 30 and 31, have been represented. These solenoid valves are connected to the abovementioned control system 19, not shown on this figure.

The compounds intended to react together are for example ammonium carbonate and calcium chloride.

### Examples

The following formulas are prepared (proportions expressed by weight relative to the total weight of the mixture).

### Formula 1:

| | |
|---|---|
| Alumina 400 µm (GF09529768 from the company Aldrich) | 20% |
| Water | 80% |

### Formula 2:

| | |
|---|---|
| Alumina 45 µm (GF18024511 from the company Aldrich) | 20% |
| Water | 80% |

### Formula 3:

| | |
|---|---|
| Alumina 0.1 µm (GF29729650 from the company Aldrich) | 20% |
| Water | 80% |

### Formula 4:

| | |
|---|---|
| KCl (average size of 420 µm) | 20% |
| Ethanol | 80% |

### Formula 5:

| | |
|---|---|
| Expanded perlite 25 microns (Optimat 2550 OR from World Minerals-Imerys) | 4% |
| Water | 96% |

### Example 1

A device according to the invention, as illustrated in Figure 2, is used with a flow rate of 2 L/min. This device has a pump delivering the composition under a pressure of 10 bar, two single-orifice nozzles of circular cross-section, oriented towards one another at 90°, a grid upstream of the nozzles serving as a filter for preventing particles of more than 500 µm from reaching the nozzles, and a reservoir.

The device is supplied with water. The formulas mentioned above are introduced into the reservoir. The device allows an injection at 30% by weight of the formulas into the flow of water.

Three heads of hair having previously undergone two successive bleaching operations (length 30 cm caucasian initially chestnut brown) are treated for 10 seconds.

On each of the heads of hair, the device is used for 20 seconds while taking care to move the hand piece so as to treat the whole of one half-surface.

### Comparative Example 1:

The settings of the device of Example 1 are changed: The supply of the pump is turned off. In so doing, the pressure is limited to 3.4 bar, which is the water supply pressure. The two-nozzle head is replaced with a single nozzle, with an orifice 5 mm in diameter. In so doing, the flow rate is 12 L/min.

The device is used with these new settings and the same formulas. The other half of each of the heads of hair is targeted.

At the end of treatment, the heads of hair are rinsed with 2 l of water applied with a flow rate of 12 L/min.

Each head of hair is shampooed and the feel is noted on wet hair and on dry hair. A difference in quality of feel is noted on wet hair in favour of the first setting. A better sheen (on dry hair) is also noted for Formulas 2 and 3.

### Example 2:

The same settings are performed for treating a scalp with dandruff. In the case of Formulas 1 and 2, an improvement in the dandruff state is noted in the case of the device according to the invention, compared with the starting state. The dandruff flakes are, according to the visual observation, to a large extent removed. This is due in particular to the size of the particles, which is preferentially between 10 and 300 microns for a treatment of a dandruff state of the scalp. Conversely, when the same composition, sent in a conventional stream (10 L/min), is used, no improvement in the dandruff state is observed.

### Example 3:

Formula 1 is introduced into a 2-litre reservoir. The device is used by supplying it by means of the reservoir, as in the example of Figure 1. The pump supply tube is immersed in the reservoir. The hand piece is used for treating the head of hair, then the body, making it possible to exfoliate the front and back of the body without difficulty.

### Example 4:

Formulas 1 and 2 are mixed in a ratio of 50/50 by volume. The whole mixture is introduced into a 2-litre tank. The device is used by supplying it with this formula, as for the previous example, the pump supply tube being immersed in the tank. The tank is stirred, then the stirring is stopped and its content is dispensed onto the head of hair. Firstly, the large particles are dispensed. The individual feels the activation and can indicate whether they wish to stop or to continue. If the device is left to continue dispensing the composition, the device sends the finer particles, then water, because of the settling which occurs in the tank.

### Example 5:

Formula 4 is used in the device according to the invention of Example 1. A head of hair is treated in order to activate the scalp. The treatment lasts approximately 10 minutes. After a pause of 5 minutes, the head of hair is rinsed for 1 minute with a supply without Formula 4. By virtue of the invention, the water consumption is limited (22 l). Finally, the scalp and the head of hair contain no powder residue.

### Example 6:

Formula 5 is used in the device according to the invention of Example 1. A head of hair is treated in order to activate the scalp. The treatment lasts approximately 10 minutes. After a pause of 5 minutes, the head of hair is placed in a sink and then rinsed for 5 minutes with a supply without Formula 4 while at the same time keeping the sink filling. The hair is left to bathe, leaving the particles to rise to the surface. Once the head of hair has been removed from the sink, the particles floating at the surface are then recovered. The scalp and the head of hair contain no powder residue. In addition, such a treatment is economical.

## Claims

1. Process for subjecting a surface of the skin or of the hair to an abrasive and/or stimulating action, comprising projecting onto said surface a flow of at least one composition comprising a vector liquid and solid particles, this flow being generated from the collision of at least two jets generated by at least two nozzles (13) of a dispensing device (10), the nozzles being oriented with an angle of between 30 and 120° with respect to one another such that their jets encounter one another, at least one nozzle (13) being supplied with a pressure of at least 4 bar and with a vector-liquid flow rate of less than or equal to 10 L/min,
the dispensing device (10) comprising a hand piece (11) having a cavity (89) opened to the outside, within which the jets emitted by the nozzles (13) encounter one another.

2. Process according to Claim 1, the vector liquid comprising water, better still being composed predominantly of water.

3. Process according to either of Claims 1 and 2, the flow rate being between 0.4 L/min and 4 L/min, better still between 1 and 4 L/min and/or the pressure being greater than or equal to 6 bar, better still between 6 and 20 bar, even better still between 8 and 20 bar.

4. Process according to any one of the preceding claims, the nozzles (13) being oriented with an angle of about 90° with respect to one another.

5. Process according to any one of the preceding claims, the solid particles being present in the composition upstream of the nozzle(s) (13) in a concentration by weight of greater than or equal to 0.5% relative to the total weight of the composition, better still between 0.5% and 5% or 5% and 25% the particles having especially a hardness on the Mohs scale of greater than or equal to 3, the particles being in particular chosen from powders of alumina, of silica, of aluminosilicates, of carbonates, or of a material coated with a silica, an alumina or an aluminosilicate, and mixtures thereof.

6. Process according to any one of the preceding claims, the particle speed at the moment they impact said surface being greater than or equal to 8 m/s and preferably greater than 15 m/s and/or the particle size being between 0.1 and 500 microns and/or the particles having a density of less than 1 g/cm³, preferably being chosen from cellulose aerogels and powders.

7. Process according to any one of the preceding claims, said surface being a surface of the hair, the treatment in particular aiming to remove the edges of flaking and to improve the sheen of the hair, to sensitize the surface of the hair, and/or to degrease the hair.

8. Process according to any one of Claims 1 to 5, said surface being a skin surface, in particular a scalp surface, the treatment aiming in particular to exfoliate or remove the dandruff, to stimulate the skin, in particular of the scalp, for the purposes of massaging or relaxing, and/or to treat acneic healthy skin.

9. Process according to any one of the preceding claims, comprising the step consisting in rinsing said surface with the same device as that used for projecting the composition, by projecting water without said solid particles and/or water with a surfactant or a compound capable of dissolving the particles.

10. Process according to any one of the preceding claims, in which said particles are suspended by mechanical stirring in a reservoir and they are left to settle, and, during the settling, the composition is suctioned from this reservoir so as to allow the nature and/or the concentration of the particles present in the flow distributed to vary due to the settling taking place in the reservoir, especially at least two powders of different densities, in particular calcium carbonate and an aluminosilicate, are mixed in said reservoir.

11. Process according to any one of the preceding claims, the particles being water-soluble and taken from a reservoir (24) where they are in suspension in a liquid in which they are not soluble, this liquid being in particular ethanol and/or the particles being generated at the time of use by bringing together two compounds which react together to form solid particles, these compounds being in particular injected into the water supplying a pump (14) of the device upstream of said pump.

12. Device (10) for treating the skin or the hair, in particular for the implementation of the process as defined in any one of the preceding claims, comprising:
- a device for dispensing a liquid cosmetic composition comprising a vector liquid and solid particles entrained by this liquid, this device comprising at least two nozzles (13) oriented such that their jets encounter one another to generate a flow that is dispensed on the skin or hair, the axes (X₁, X₂) of the nozzles making an angle between them of between 60° and 120°, in particular of about 90° at least one nozzle being supplied with the vector liquid at a pressure of at least 4 bar so as to deliver a flow of composition at a flow rate of less than or equal to 10 L/min, the dispensing device (10) comprising a hand piece (11) having a cavity (89) opened to the outside, within which the jets emitted by the nozzles (13) encounter one another, and
- a reservoir (16; 24) containing said composition or the solid particles to be mixed with the vector liquid, or at least one reservoir (30; 31) containing a compound capable of generating, by reacting with at least one other compound, said solid particles.

13. Device according to the preceding claim, comprising a pump (14) connected to said at least one nozzle (13) and/or comprising a stirrer (22) for suspending said particles in the reservoir, the device being in particular arranged to remove the composition to be dispensed by suctioning from this reservoir (16), the suctioning being carried out at a distance from the bottom of the reservoir.

14. Device according to either of Claims 12 and 13, comprising two reservoirs (30, 31) containing compounds which react together, and a system for injecting (26, 27) these compounds so as to allow them to mix with the vector liquid and to react, the reaction of the compounds producing solid particles.

## Patentansprüche

1. Verfahren zum Aussetzen einer Oberfläche der Haut oder der Haare einer abrasiven und/oder stimulierenden Wirkung, umfassend Projizieren auf diese Oberfläche einen Strom mindestens einer Zusammensetzung, die eine Vektorflüssigkeit und feste Partikel umfasst, wobei dieser Strom durch die Kollision von mindestens zwei Strahlen erzeugt wird, die von mindestens zwei Düsen (13) einer Abgabevorrichtung (10) erzeugt werden, wobei die Düsen in einem Winkel zwischen 30 und 120° zueinander ausgerichtet sind, so dass ihre Strahlen aufeinander treffen, wobei mindestens eine Düse (13) mit einem Druck von mindestens 4 bar und mit einem Vektor-Flüssigkeitsdurchflussrate von weniger als oder gleich 10 l/min versorgt wird,
wobei die Abgabevorrichtung (10) ein Handstück (11) umfasst, das einen nach außen geöffneten Hohlraum (89) aufweist, in dem die von den Düsen (13) abgegebenen Strahlen aufeinander treffen.

2. Verfahren nach Anspruch 1, wobei die Vektorflüssigkeit Wasser enthält, besser noch überwiegend aus Wasser besteht.

3. Verfahren nach einem der Ansprüche 1 und 2, wobei die Durchflussrate zwischen 0,4 l/min und 4 l/min, besser noch zwischen 1 und 4 l/min liegt und/oder der Druck größer oder gleich 6 bar, besser noch zwischen 6 und 20 bar, noch besser zwischen 8 und 20 bar ist.

4. Verfahren nach einem der vorhergehenden Ansprüche, wobei die Düsen (13) in einem Winkel von etwa 90° zueinander ausgerichtet sind.

5. Verfahren nach einem der vorhergehenden Ansprüche, wobei die festen Partikel in der Zusammensetzung stromaufwärts der Düse(n) (13) in einer Gewichtskonzentration von größer als oder gleich 0,5 %, bezogen auf das Gesamtgewicht der Zusammensetzung, vorhanden sind, besser noch zwischen 0.5 % und 5 % oder 5 % und 25 %, wobei die Partikel insbesondere eine Härte auf der Mohs-Skala von größer oder gleich 3 aufweisen, wobei die Partikel insbesondere aus den Pulvern von Aluminiumoxid, von Siliciumdioxid, von Aluminosilicaten, von Carbonaten oder von einem mit einem Siliciumdioxid, einem Aluminiumoxid oder einem Aluminosilicat beschichteten Material sowie deren Mischungen ausgewählt sind.

6. Verfahren nach einem der vorhergehenden Ansprüche, wobei die Partikelgeschwindigkeit zum Zeitpunkt ihres Auftreffens auf die Oberfläche größer oder gleich 8 m/s und vorzugsweise größer als 15 m/s ist und/oder die Partikelgröße zwischen 0,1 und 500 Mikrometer liegt und/oder die Partikel eine Dichte von weniger als 1 g/cm³ aufweisen, wobei sie vorzugsweise aus Zellulose-Aerogelen und -Pulvern ausgewählt sind.

7. Verfahren nach einem der vorhergehenden Ansprüche, wobei die Oberfläche eine Oberfläche der Haare ist und die Behandlung insbesondere darauf abzielt, die Schuppenränder zu entfernen und den Glanz des Haares zu verbessern, die Haaroberfläche zu sensibilisieren und/oder das Haar zu entfetten.

8. Verfahren nach einem der Ansprüche 1 bis 5, wobei die Oberfläche eine Hautoberfläche, insbesondere eine Kopfhautoberfläche, ist und die Behandlung insbesondere darauf abzielt, die Schuppen abzuschälen oder zu entfernen, die Haut, insbesondere die Kopfhaut, zum Zwecke der Massage oder Entspannung zu stimulieren und/oder akneartige gesunde Haut zu behandeln.

9. Verfahren nach einem der vorhergehenden Ansprüche, das den Schritt umfasst, der darin besteht, die Oberfläche mit der gleichen Vorrichtung zu spülen, die für das Projizieren der Zusammensetzung verwendet wird, indem Wasser ohne die festen Partikel und/oder Wasser mit einem Tensid oder einer Verbindung, die die Partikel auflösen kann, projiziert wird.

10. Verfahren nach einem der vorhergehenden Ansprüche, bei dem die Partikel durch mechanisches Rühren in einem Behälter suspendiert werden und diese absetzen lässt, und bei dem während des Absetzens die Zusammensetzung aus diesem Behälter gesaugt wird, so dass die Art und/oder die Konzentration der in dem verteilten Strom vorhandenen Partikel aufgrund des im Behälter stattfindenden Absetzens variieren kann, wobei insbesondere mindestens zwei Pulver mit unterschiedlicher Dichte, insbesondere Calciumcarbonat und ein Aluminosilikat, in dem Behälter gemischt werden.

11. Verfahren nach einem der vorhergehenden Ansprüche, wobei die Partikel wasserlöslich sind und aus einem Behälter (24) entnommen werden, wo sie in einer Flüssigkeit suspendiert sind, in der sie nicht löslich sind, wobei diese Flüssigkeit insbesondere Ethanol ist, und/oder die Partikel zum Zeitpunkt der Verwendung durch Zusammenbringen von zwei Verbindungen erzeugt werden, die miteinander reagieren, um feste Partikel zu bilden, wobei diese Verbindungen insbesondere in das Wasser eingespritzt werden, das eine Pumpe (14) der Vorrichtung stromaufwärts der Pumpe versorgt.

12. Vorrichtung (10) zur Behandlung der Haut oder der Haare, insbesondere zur Durchführung des Verfahrens nach einem der vorhergehenden Ansprüche, umfassend:
- Vorrichtung zur Abgabe einer flüssigen kosmetischen Zusammensetzung, die eine Vektorflüssigkeit und von dieser Flüssigkeit mitgenommene feste Partikel umfasst, wobei die Vorrichtung mindestens zwei Düsen (13) umfasst, die so ausgerichtet sind, dass ihre Strahlen aufeinander treffen, um einen auf die Haut oder die Haare abgegebenen Strom zu erzeugen, wobei die Achsen (X₁, X₂ ) der Düsen untereinander einen Winkel zwischen 60° und 120° bilden, insbesondere von etwa 90°, wobei mindestens eine Düse mit der Vektorflüssigkeit mit einem Druck von mindestens 4 bar versorgt wird, um einen Strom der Zusammensetzung mit einer Durchflussrate von weniger als oder gleich 10 l/min abzugeben, wobei die Abgabevorrichtung (10) ein Handstück (11) umfasst, das einen nach außen geöffneten Hohlraum (89) aufweist, in dem die von den Düsen (13) abgegebenen Strahlen aufeinander treffen, und
- einen Behälter (16; 24), der die Zusammensetzung oder die mit der Vektorflüssigkeit zu mischenden festen Partikel enthält, oder mindestens einen Behälter (30; 31), der eine Verbindung enthält, die durch Reaktion mit mindestens einer anderen Verbindung die festen Partikel erzeugen kann.

13. Vorrichtung nach dem vorhergehenden Anspruch, umfassend eine Pumpe (14), die mit der mindestens einen Düse (13) verbunden ist, und/oder einen Rührer (22) zum Suspendieren der Partikel in dem Behälter, wobei die Vorrichtung insbesondere so angeordnet ist, dass sie die abzugebende Zusammensetzung durch Absaugen aus diesem Behälter (16) entfernt, wobei das Absaugen in einem Abstand vom Boden des Behälters erfolgt.

14. Vorrichtung nach einem der Ansprüche 12 und 13, umfassend zwei Behälter (30, 31), die miteinander reagierende Verbindungen enthalten, und ein System zum Einspritzen (26, 27) dieser Verbindungen, damit sie sich mit der Vektorflüssigkeit vermischen und reagieren können, wobei die Reaktion der Verbindungen feste Partikel erzeugt.

## Revendications

1. Procédé pour soumettre une surface de la peau ou des cheveux à une action abrasive et/ou stimulante, comprenant la projection sur ladite surface d'un flux d'au moins une composition comprenant un liquide vecteur et des particules solides, ce flux étant généré à partir de la collision d'au moins deux jets générés par au moins deux buses (13) d'un dispositif de distribution (10), les buses étant orientées avec un angle compris entre 30 et 120° l'une par rapport à l'autre de sorte que leurs jets se rencontrent, au moins une buse (13) étant fournie avec une pression d'au moins 4 bars et avec un débit en liquide vecteur inférieur ou égal à 10 L/min,
le dispositif de distribution (10) comprenant une pièce à main (11) ayant une cavité (89) ouverte vers l'extérieur, au sein de laquelle les jets émis par les buses (13) se rencontrent.

2. Procédé selon la revendication 1, le liquide vecteur comprenant de l'eau, mieux se composant majoritairement d'eau.

3. Procédé selon l'une des revendications 1 et 2, le débit étant compris entre 0,4 L/min et 4 L/min, mieux entre 1 et 4 L/min et/ou la pression étant supérieure ou égale à 6 bars, mieux comprise entre 6 et 20 bars, encore mieux entre 8 et 20 bars.

4. Procédé selon l'une quelconque des revendications précédentes, les buses (13) étant orientées avec un angle d'environ 90° l'une par rapport à l'autre.

5. Procédé selon l'une quelconque des revendications précédentes, les particules solides étant présentes dans la composition en amont de la buse ou des buses (13) en une concentration en poids supérieure ou égale à 0,5 % par rapport au poids total de la composition, mieux entre 0,5 et 5 % ou 5 et 25 %, les particules ayant en particulier une dureté sur l'échelle de Mohs supérieure ou égale à 3, les particules étant notamment choisies parmi les poudres d'alumine, de silice, d'aluminosilicates, de carbonates, ou d'un matériau enrobé par une silice, une alumine ou un aluminosilicate, et leurs mélanges.

6. Procédé selon l'une quelconque des revendications précédentes, la vitesse des particules au moment où elles impactent ladite surface étant supérieure ou égale à 8 m/s et de préférence supérieure à 15 m/s et/ou la taille des particules étant comprise entre 0,1 et 500 microns et/ou les particules ayant une densité inférieure à 1 g/cm³, de préférence étant choisies parmi les aérogels et les poudres de cellulose.

7. Procédé selon l'une quelconque des revendications précédentes, ladite surface étant une surface des cheveux, le traitement visant notamment à retirer les bords d'écailles et à améliorer la brillance du cheveu, réactiver la surface du cheveu, et/ou dégraisser le cheveu.

8. Procédé selon l'une quelconque des revendications 1 à 5, ladite surface étant une surface de peau, notamment une surface de cuir chevelu, le traitement visant notamment à exfolier ou éliminer les pellicules, à stimuler la peau, notamment le cuir chevelu, à des fins de massage ou de relaxation, et/ou à traiter une peau saine acnéique.

9. Procédé selon l'une quelconque des revendications précédentes, comprenant l'étape consistant à rincer ladite surface avec le même dispositif que celui utilisé pour projeter la composition, en projetant de l'eau sans lesdites particules solides et/ou de l'eau avec un tensioactif ou un composé apte à dissoudre les particules.

10. Procédé selon l'une quelconque des revendications précédentes, dans lequel lesdites particules sont suspendues par agitation mécanique dans un réservoir et on les laisse décanter, et pendant la décantation, la composition est aspirée de ce réservoir de manière à permettre à la nature et/ou la concentration des particules présentes dans le flux distribué de changer du fait de la décantation qui a lieu dans le réservoir, en particulier au moins deux poudres de différentes densités, notamment du carbonate de calcium et un aluminosilicate, sont mélangées dans ledit réservoir.

11. Procédé selon l'une quelconque des revendications précédentes, les particules étant solubles dans l'eau et prélevées d'un réservoir (24) où elles sont en suspension dans un liquide dans lequel elles ne sont pas solubles, ce liquide étant notamment de l'éthanol et/ou les particules étant générées au moment de l'utilisation en mettant en présence deux composés qui réagissent ensemble pour former des particules solides, ces composés étant notamment injectés dans l'eau alimentant une pompe (14) du dispositif en amont de ladite pompe.

12. Dispositif (10) pour traiter la peau ou les cheveux, notamment pour la mise en œuvre du procédé tel que défini dans l'une quelconque des revendications précédentes, comprenant :
- un dispositif pour distribuer une composition cosmétique liquide comprenant un liquide vecteur et des particules solides entraînées par ce liquide, ce dispositif comprenant au moins deux buses (13) orientées de sorte que leurs jets se rencontrent pour générer un flux qui est distribué sur la peau ou les cheveux, les axes (X₁, X₂) des buses faisant un angle entre elles compris entre 60° et 120°, notamment d'environ 90°, au moins une buse étant fournie avec le liquide vecteur à une pression d'au moins 4 bars de manière à distribuer un flux de composition à un débit inférieur ou égal à 10 L/min, le dispositif de distribution (10) comprenant une pièce à main (11) ayant une cavité (89) ouverte vers l'extérieur, au sein de laquelle les jets émis par les buses (13) se rencontrent, et
- un réservoir (16 ; 24) contenant ladite composition ou les particules solides à mélanger avec le liquide vecteur, ou au moins un réservoir (30 ; 31) contenant un composé apte à générer, en réagissant avec au moins un autre composé, lesdites particules solides.

13. Dispositif selon la revendication précédente, comprenant une pompe (14) reliée à ladite au moins une buse (13) et/ou comprenant un agitateur (22) pour suspendre lesdites particules dans le réservoir, le dispositif étant notamment agencé pour retirer la composition à distribuer par aspiration de ce réservoir (16), l'aspiration étant réalisée à une distance du fond du réservoir.

14. Dispositif selon l'une des revendications 12 et 13, comprenant deux réservoirs (30, 31) contenant des composés réagissant ensemble, et un système d'injection (26, 27) de ces composés de manière à leur permettre de se mélanger au liquide vecteur et de réagir, la réaction des composés produisant des particules solides.
